(19) Europäisches Patentamt
European Patent Office
Office européen des brevets

(11) Veröffentlichungsnummer: **0 074 559**
**B1**

# (12) EUROPÄISCHE PATENTSCHRIFT

(45) Veröffentlichungstag der Patentschrift: **23.01.85**

(21) Anmeldenummer: **82108023.1**

(22) Anmeldetag: **01.09.82**

(51) Int. Cl.[4]: **C 07 C 103/44**, C 07 C 103/64, C 07 D 295/18, C 09 B 67/20, C 09 D 5/00, C 09 D 11/02

(54) **Beta-Ketoamide und diese Amide enthaltende Pigmentzubereitungen.**

(30) Priorität: **10.09.81 DE 3135848**

(43) Veröffentlichungstag der Anmeldung: **23.03.83 Patentblatt 83/12**

(45) Bekanntmachung des Hinweises auf die Patenterteilung: **23.01.85 Patentblatt 85/4**

(84) Benannte Vertragsstaaten: **CH DE FR GB IT LI**

(56) Entgegenhaltungen:
**CH - A - 505 788**
**DE - A - 1 571 897**

(73) Patentinhaber: **BASF Aktiengesellschaft, *Carl-Bosch-Strasse 38, D-6700 Ludwigshafen (DE)***

(72) Erfinder: **Bock, Gustav, Dr., Waldstrasse 16, D-6730 Neustadt (DE)**
Erfinder: **Frey, Guenter, Dr., Mainstrasse 13, D-6701 Dannstadt-Schauernheim (DE)**
Erfinder: **Jettmar, Werner, Dr., Damaschkering 60, D-6800 Mannheim (DE)**
Erfinder: **Radtke, Volker, Dr., Barbarossastrasse 4, D-6733 Hassloch (DE)**
Erfinder: **Sappok, Reinhard, Dr., 2446 Sunset Bluff Drive, Holland, Mich. 49423 (US)**

**EP 0 074 559 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue β-Ketoamide, deren Salze und Pigmentzubereitungen, die diese β-Ketoamide enthalten.

Aus der DE-A 1 571 897 sind Azoacylacetamide der allgemeinen Formel

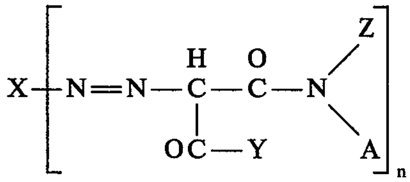

bekannt, in der X für einen gegebenenfalls substituierten Arylrest oder heterocyclischen Rest, Y für Alkyl oder für gegebenenfalls substituiertes Aryl, Z für Wasserstoff oder ein gegebenenfalls substituiertes Alkyl und A für Alkenyl, gegebenenfalls substituiertes Alkyl mit mehr als 10 C-Atomen oder für ein eine sekundäre oder tertiäre Aminogruppe tragendes Alkyl und n für eine ganze Zahl stehen.

Die Azoacylacetamide der DE-A sind in organischen Lösungsmitteln, wie sie zur Herstellung von Druckfarben und Lacken verwendet werden, leicht löslich und sollen in diesen Lösungsmitteln sehr wirksame Dispergiermittel bzw. Entflockungsmittel, insbesondere für Azoacylacetarylamidpigmente und andere Pigmente sein.

Nach den Angaben in der Beschreibung und in den Beispielen leitet sich der Rest X von Diazokomponenten ab, wie sie bei der Herstellung von Azopigmenten verwendet werden, z. B. 3,3′-Dichlorbenzidin und durch Methyl oder Chlor substituierte Nitraniline. Nach den Angaben in den Beispielen werden Azopigmente durch die Gegenwart dieser Azoacylacetamide gegen Flockung stabilisiert. D. h. Stabilisierungsmittel und das zu stabilisierende Pigment sind analog aufgebaut und weisen in einem Teil des Moleküls die gleichen oder sehr ähnliche Gruppen auf.

Die neuen β-Ketoamide haben die allgemeine Formel

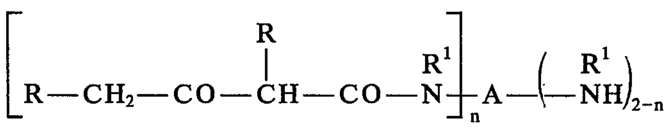 (I)

in der R für $C_8$– bis $C_{18}$-Alkyl oder $C_8$– bis $C_{18}$-Alkenyl, $R^1$ für Wasserstoff und –A– für 1,5-Naphthylen, 1,8-Naphthylen, 1,3-Phenylen, 1,4-Phenylen, für gegebenenfalls in 3,3′- oder in 2,2′-Stellung durch Chlor, Methyl oder Methoxy substituiertes 4,4′-Diphenylen, 4,4′-Diphenylenether, für gegebenenfalls in 3,3′-Stellung durch Isopropyl oder Methoxy und gegebenenfalls in 5,5′-Stellung durch Isopropyl substituiertes 4,4′-Diphenylenmethan oder 4,4′-Dicyclohexylmethan für gegebenenfalls in 3,3′-Stellung durch Isopropyl oder Methoxy substituiertes 4-Cyclohexylen-4′-phenylenmethan, für 2,2-Bis(phenylen-4′)-propan, 2,2-Bis(cyclohexylen-4′)propan, 2-(Cyclohexylen-4′)-2-(phenylen-4″)-propan, 1,2-Cyclohexylen, 1,3-Bismethylenphenyl, 4,4′-Diphenylenamin, Di-(3-methylphenylen-4)-methan, 3-Methylcyclohexylen-4-3′-methylphenylen-4′-methan, Bis-(3-methylcyclohexylen-4)-methan oder

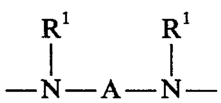 für 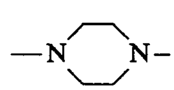

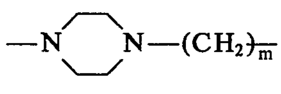

oder

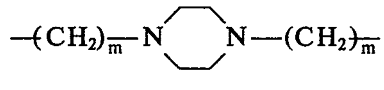

n für 1 oder 2 und

m für 2 oder 3 stehen

und wenn n = 1 ist, können die Mono-β-ketoamide auch in Form der Salze von $C_{10}$- bis $C_{20}$-Alkansäuren, $C_{10}$- bis $C_{20}$-Alkensäuren, $C_{10}$- bis $C_{20}$-Alkan- oder -Alkensulfonsäuren oder $C_1$- bis $C_{20}$-Alkylbenzolsulfonsäuren vorliegen.

Die Verbindungen (I) und deren Salze geben mit Pigmenten, vorzugsweise mit organischen Pigmenten, beim Einarbeiten in Bindemittellösungen Druck- und Lackfarben mit hervorragenden coloristischen Eigenschaften. Man erhält farbstarke Druck- und Lackfarben, die sehr gutes Fließverhalten aufweisen und die Färbungen mit hohem Glanz liefern. Dies gilt sowohl für lufttrocknende Lacke als auch für Einbrennlacke.

Dementsprechend betrifft die Erfindung außerdem neue Pigmentzubereitungen, die a) ein feinteiliges organisches Pigment, das sich vom Anthrachinon oder davon abgeleiteten kondensierten Ringsystemen, von der Perylen-3,4,9,10-tetracarbonsäure, von Chinophthalonen, Dioxazinen, Chinacridon oder vom Phthalocyanin ableitet, und b) mindestens ein Ketoamid der Formel I und/oder deren Salze enthalten.

Die Reste R in (I) sind solche mit 8 bis 18 C-Atomen, d. h. der Gruppe $R{-}CH_2{-}CO{-}$ liegen $C_{10}$- bis $C_{20}$-Alkan- oder -Alkensäuren zugrunde.

Bevorzugt sind $\beta$-Ketoamide der Formel I mit R = $C_{10}$- bis $C_{16}$-Alkyl oder -Alkenyl, insbesondere solche, bei denen sich der Rest $R{-}ClH_2{-}CO{-}$ von der Laurinsäure, der Palmitinsäure, der Stearinsäure oder der Ölsäure ableitet, d. h. $R = C_{10}H_{21}$, $C_{14}H_{29}$, $C_{16}H_{33}$ oder $C_{16}H_{31}$ ist. Beide R können gleich oder verschieden sein.

Bevorzugte Salze von $\beta$-Ketoamiden der Formel I (n = 1) sind solche von $C_{12}$- bis $C_{20}$-Alkan-/-Alkensäuren und $C_8$- bis $C_{20}$-Alkylbenzolsulfonsäuren wie Palmitinsäure, Stearinsäure, Ölsäure, 4-Nonylbenzolsulfonsäure, 4-Dodecylbenzolsulfonsäure, 4-Hexadecylbenzolsulfonsäure.

Für $-A-$ ($R^1 = H$) sind zu nennen:

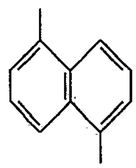 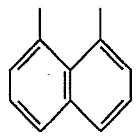 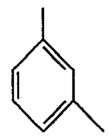 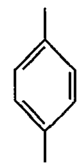

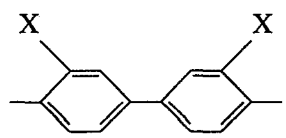 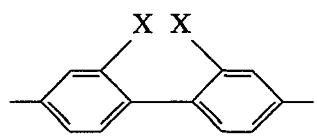

und

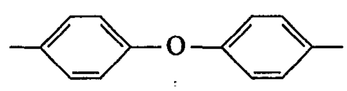

mit X = $-H$, $-Cl$, $-CH_3$ oder $-OCH_3$

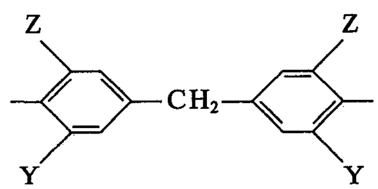 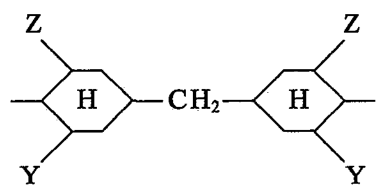

und

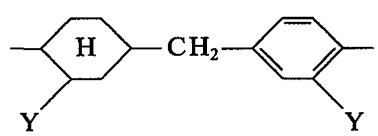

mit Y = $-H$, $-OCH_3$ oder $-CH(CH_3)_2$ und
Z = H oder $-CH(CH_3)_2$,

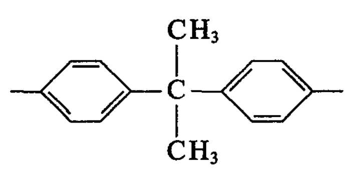 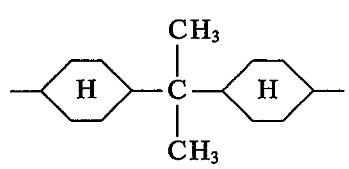

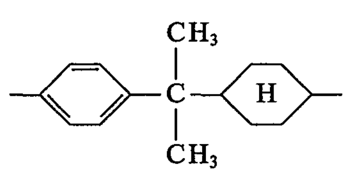 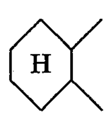

und

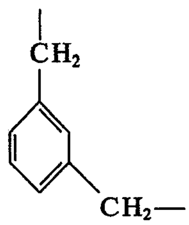

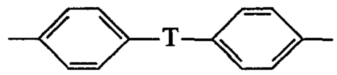

mit T = –O– oder $\overset{H}{-N-}$.

Für

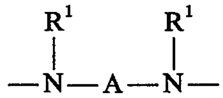

kommen in Betracht:

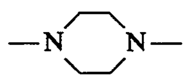

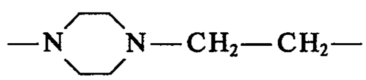

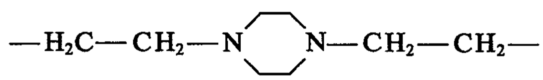

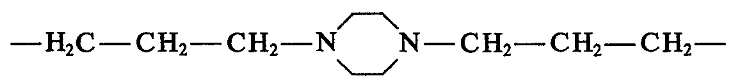

und

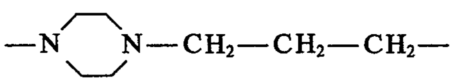

Bevorzugt sind β-Ketoamide der Formel I, in denen $R^1$ = H und –A– 1,5-Naphthylen, gegebenenfalls in 3,3'- oder in 2,2'-Stellung durch Chlor, Methyl oder Methoxy substituiertes Diphenylen-4,4', Di(phenylen-4)-methan, 2,2-Bis(phenylen-4')-propan, Di-(3-methylphenylen-4)-methan, 3-Methyl-cyclohexylen-4-3'-methylphenylen-4'-methan, Bis-(cyclohexylen-4)-methan, Bis-(3-methyl-cyclohexylen-4)-methan, 2,2-Bis(cyclohexylen-4')-propan, 4,4'-Diphenylether oder

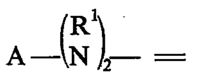 = 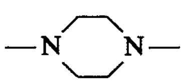 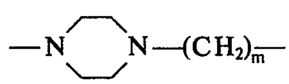

oder

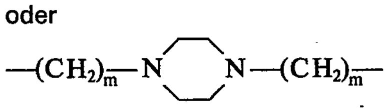

mit m = 2 oder 3

ist, da diese Ketoamide Druck- und Lackfarben besonders vorteilhafte Fließeigenschaften und den mit diesen Farben erhaltenen Färbungen besonders hohen Glanz verleihen.

Ganz besonders bevorzugt sind Ketoamide (I), in denen

—A— und 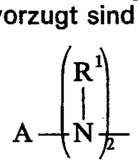

die vorstehend angegebene Bedeutung haben und R für die oben angegebenen bevorzugten Gruppen steht, und – wenn n = 1 ist – deren Salze mit $C_{10}$- bis $C_{20}$- Alkansäuren, $C_{10}$- bis $C_{20}$-Alkensäuren oder mit $C_8$- bis $C_{20}$-Alkylbenzolsulfonsäuren.

Die β-Ketoamide (I) werden in an sich bekannter Weise durch Umsetzen der Amine

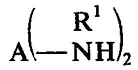

(II)

mit den entsprechenden Diketenen

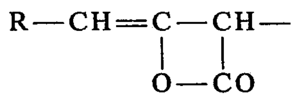

(III)

im Verhältnis 1 : 1 bis 1 : 2 Mol hergestellt. Im ersteren Fall erhält man (I) mit n = 1.

Die zur Umsetzung benötigten Amine (II) sind bekannt, desgleichen die Diketene der Formel III. Eine Herstellung von Diketenen (III) ist in der DE-OS 2 927 118 beschrieben.

Die Umsetzung erfolgt in Gegenwart von inerten organischen Flüssigkeiten, wie Benzolkohlenwasserstoffen, aliphatischen oder cycloaliphatischen Kohlenwasserstoffen oder in der Schmelze des Amins. Die Umsetzung erfolgt im allgemeinen bei Temperaturen von 50 bis 200, vorzugsweise von 80 bis 120°C.

Nach der Umsetzung wird die organische Flüssigkeit gegebenenfalls entfernt.

Einzelheiten zur Herstellung von (I) können den Beispielen entnommen werden.

Die Pigmentzubereitungen enthalten, bezogen auf Pigment (a), in der Regel 2 bis 20 Gew.-% an (I). Vorzugsweise wendet man 5 bis 15 Gew.-% (I), bezogen auf (a), an.

Bevorzugt sind Zubereitungen, die β-Ketoamide I enthalten, in denen $R^1$ = H und —A— 1,5-Naphthylen, gegebenenfalls in 3,3'- oder in 2,2'-Stellung durch Chlor, Methyl oder Methoxy substituiertes Diphenylen-4,4', Di(phenylen-4)-methan, 2,2-Bis(phenylen-4')-propan, Di-(3-methylphenylen-4)-methan, 3-Methyl-cyclohexylen-4-3'-methylphenylen-4'-methan, Bis-(cyclohexan-4)-methan, Bis-(3-methyl-cyclohexylen-4)-methan, 2,2-Bis(cyclohexylen-4')-propan, 4,4'-Diphenylether oder

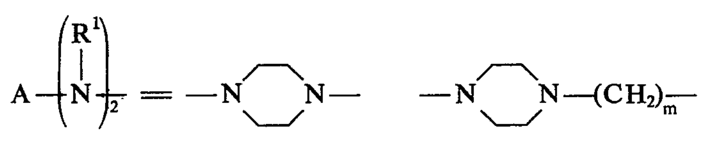

oder

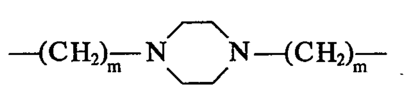

mit m = 2 oder 3 und R = $C_{10}$- bis $C_{16}$-Alkyl oder $C_{10}$- bis $C_{16}$-Alkenyl sind.

Besonders bevorzugt sind Zubereitungen, die β-Ketoamide der Formel I enthalten, in der —A— oder $A(NR^1)_2$— die vorstehend angegebene Bedeutung haben und R—$CH_2$—CO— ein Rest der Laurin-, Palmitin-, Stearin- oder Ölsäure ist.

Die Zubereitungen können durch Mischen des feinteiligen Pigments (a) mit (I) in Form eines Pulvers, z. B. durch Mahlen oder im Mischer hergestellt werden. Die Zubereitung kann auch durch Formieren des Rohpigments in Gegenwart von (I) oder durch gemeinsames Anreiben von Pigment und (I) in für Druck- oder Lackfarben geeigneten Bindemitteln oder Bindemittellösungen hergestellt werden. In allen Fällen erzielt man die gleiche Wirkung.

Als organische Pigmente kommen solche in Betracht, die sich von Anthrachinon und davon abgeleiteten kondensierten Ringsystemen, von der Perylen-3,4,9,10-tetracarbonsäure, von Chinophthalonen, Dioxazinen, Chinacridon und von Phthalocyanin ableiten. Im einzelnen sind z. B. zu nennen: Flavanthron, Indanthron und dessen Chlorierungsprodukte, Pyranthron, Dichlorpyranthron, Monobrom- und Dibrom-dichlorpyranthron, Tetrabrompyranthron, Perylen-3,4,9,10-tetracarbonsäurediimide, wobei die Imidgruppen unsubstituiert, durch $C_1$- bis $C_3$-Alkyl oder durch phenylische oder heterocyclische Reste substituiert sein können, Anthrapyrimidincarbonsäureamide, Violanthron, Isoviolanthron, Dioxazinpigmente, Kupferphthalocyanine, Polychlorkupferphthalocyanine und Polybromchlorkupferphthalocyanine mit bis zu 14 Bromatomen.

Die Pigmentzubereitungen können neben (a) und (I) gegebenenfalls noch weitere in Pimentzubereitungen übliche Mittel (c) enthalten, z. B. benetzend wirkende Mittel, entstaubend wirkende Mittel. Die Menge an diesen Mitteln wird so gering wie möglich bemessen und sollte 5 Gew.-%, bezogen auf die Zubereitung (a + I) nicht überschreiten. Vorzugsweise liegt der Anteil an (c) unterhalb von 1, insbesondere unterhalb von 0,1 Gew.-%, bezogen auf (a + I).

Die Erfindung soll durch die folgenden Beispiele weiter erläutert werden. Die Teile und Prozentangaben beziehen sich auf das Gewicht. Die Aminzahl wurde durch Titration einer Probe in Eisessig mit Perchlorsäure bestimmt.

## Beispiel 1

In einem Dreihalskolben mit Rührer, Rückflußkühler und Thermometer werden 910 Teile Bisdecyldiketen (mittleres M. 421; Gehalt: 92,5% = 842 Teile 100% = 2 Mol) und 161 Teile (1 Mol) 1,5-Diaminophthalin (98%ig) in 1500 Teilen Toluol bei 50°C unter Stickstoff gelöst. Beim Erwärmen tritt bei 60 bis 70°C Reaktion ein, wobei die Temperatur auf 90°C steigt. Die Lösung wird noch 3 Stunden bei Siedetemperatur gehalten, dann wird das Toluol unter vermindertem Druck abdestilliert.

Ausbeute: 919 Teile (= 91% der Theorie) Bis-β-ketoamid der Formel

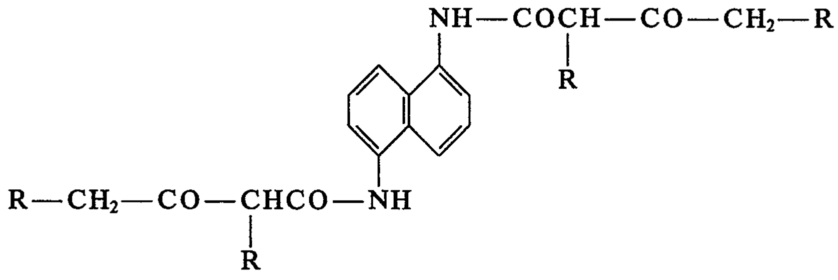

(IV)

mit R = $C_{10}H_{21}$
in Form einer Schmelze. Schmp. 124–126°C. Aminzahl: 0 (Titration mit Perschlorsäure in Eisessig). Im IR-Spektrum ist keine Diketenbande bei 1870 $cm^{-1}$ mehr vorhanden.

Analyse: $C_{58}H_{98}N_2O_4$ (M. 886)

| | | | |
|---|---|---|---|
| ber.: | C 78,56% | H 11,06% | N 3,16% |
| gef.: | C 76,90% | H 10,70% | N 3,10% |

## Beispiel 2

Es wird wie in Beispiel 1 verfahren, jedoch werden 1090 Teile Bis-$C_{16}$-alkyldiketen (mittleres M. 542; Gehalt: 99,4% = 1084 Teile 100% = 2 Mol) verwendet. Bereits beim Lösen tritt exotherme Reaktion ein. Das Gemisch wird solange bei 90°C gehalten bis die Diketenbande im IR-Spektrum bei 1870 $cm^{-1}$ verschwunden ist.

Ausbeute: 1211 Teile (= 98% d. Th.) Bis-β-ketoamid der Formel (IV) mit R = $C_{16}H_{33}$
Aminzahl: 0

Analyse: $C_{82}H_{146}N_2O_4$ (1224)

| | | | |
|---|---|---|---|
| ber.: | C 80,46 | H 12,02 | N 2,29% |
| gef.: | C 79,60 | H 12,30 | N 2,30% |

Schmp. 95–125°C.

## Beispiel 3

Es wird wie in Beispiel 2 verfahren, jedoch werden 545 Teile Bis-$C_{16}$-alkyldiketen (mittleres M. 542; Gehalt: 99,4% = 542 Teile 100% = 1 Mol) und 750 Teile Toluol verwendet.

Ausbeute: 707 Teile (99,5% d. Th.) Mono-β-ketoamid der Formel

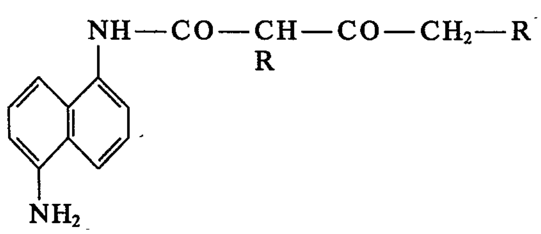

R = $C_{16}$-Alkyl
Schmp. 93°C, Aminzahl: 1,149 mmol/g

## Beispiel 4

258 Teile (1 Mol) 4,4'-Diamino-3,3'-dimethyldiphenyl (82%ig) werden in 500 Teilen Toluol gelöst und vorhandenes Wasser durch azeotrope Destillation ausgekreist (44 Teile). Zu dieser Lösung gibt man bei 80°C eine 45°C warme Lösung von 1090 Teilen Bis-$C_{16}$-alkyldiketen (mittleres M. 542; Gehalt: 99,4% ≙ 1084 Teile 100% = 2 Mol) in 1000 Teilen Toluol, wobei die Temperatur auf 88°C steigt. Nach 3 Stunden bei 90°C ist im IR-Spektrum einer Probe keine Diketenbande bei 1870 $cm^{-1}$ mehr vorhanden. Das Toluol wird unter vermindertem Druck abdestilliert.

Ausbeute: 1285 Teile (= 98% d. Th.) Bis-$\beta$-ketoamid der Formel

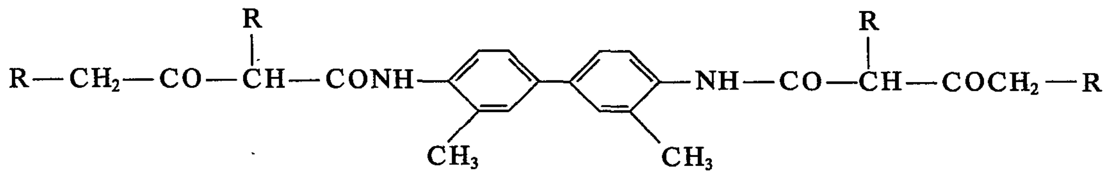 (V)

R = $C_{16}$-Alkyl

in Form einer erstarrten Schmelze.

Trockengehalt: 98,3%; Schmp. 82°C, Aminzahl: 0

Analyse: $C_{86}H_{152}N_2O_4$ (M. 1278)

| | | | |
|---|---|---|---|
| ber.: | C 80,81% | H 11,99% | N 2,19% |
| gef.: | C 80,20% | H 11,70% | N 2,10% |

## Beispiel 5

Es wird wie in Beispiel 4 verfahren, jedoch werden 1160 Teile Bis-$C_{16}$-alkenyldiketen (mittleres M. 570; Gehalt: 98,3% ≙ 1141 Teile 100% = 2 Mol) in 1800 Teilen Toluol verwendet. Die Umsetzung ist nach 8 Stunden bei 90°C beendet.

Ausbeute: 1360 Teile Bis-$\beta$-ketoamid der Formel (IV) mit R = $C_{16}H_{31}$ (= 98,3% d. Th.)
Schmp. 47 bis 49°C
Trockengehalt: 98,8%

Analyse: $C_{86}H_{148}N_2O_4$ (M. 1270)

| | | | |
|---|---|---|---|
| ber.: | C 79,80 | H 10,80 | N 2,10% |
| gef.: | C 81,33 | H 11,43 | N 2,21% |

## Beispiel 6

In einem Rührgefäß mit Rührer, Rückflußkühler und Thermometer werden unter Stickstoff 210 Teile (= 1 Mol) 4,4'-Diaminodicyclohexylmethan bei 60°C geschmolzen. In 1,5 Stunden tropft man 1090 Teile Bis-$C_{16}$-alkyldiketen (mittleres M. 542; Gehalt: 99,4% ≙ 1084 Teile 100% = 2 Mol) in Form einer Schmelze zu, wobei die Temperatur auf 100°C steigt. Die Schmelze wird 30 min bei 100°C gerührt, dann ist in einer Probe keine Diketenbande (1870 $cm^{-1}$) mehr nachweisbar.

Ausbeute: 1286 Teile (= 99,5% d. Th.) Bis-$\beta$-ketoamid der Formel

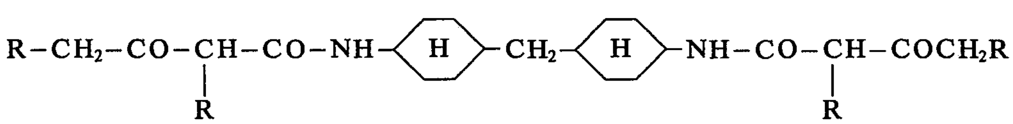

R = $C_{16}H_{33}$
Schmp. 79–81°C; Aminzahl: 0
Trockengehalt: 99,6%

Analyse $C_{85}H_{162}N_2O_4$ (M. 1276)

| | | | |
|---|---|---|---|
| ber.: | C 80,00 | H 12,80 | N 2,20% |
| gef.: | C 79,20 | H 12,30 | N 2,00% |

Beispiel 7

Es wird wie in Beispiel 6 verfahren, jedoch werden nur 545 Teile des in Beispiel 2 angegebenen Diketens (= 1 Mol) angewendet.

Ausbeute: 748 Teile (= 99% d. Th.) Mono-$\beta$-ketoamid der Formel

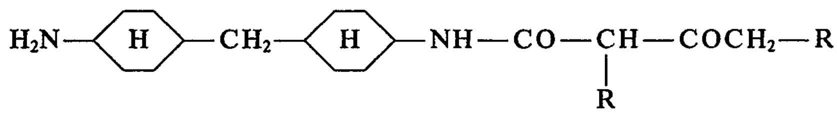

$R = C_{16}H_{33}$

Schmp. 92–94°C;
Aminzahl: 1,336 mmol/g
Trockengehalt: 99,1%.

Beispiel 8

In einem Rührgefäß (Rührer, Rückflußkühler und Thermometer) werden 86 Teile (1 Mol) Piperazin (wasserfrei) in 1000 Teilen Toluol auf 60°C erwärmt. Dazu gibt man in Portionen 1090 Teile des in Beispiel 2 verwendeten Diketens (= 2 Mol) und rührt das Reaktionsgemisch 14 Stunden zwischen 100 und 120°C. Dann ist kein Diketen mehr nachzuweisen. Das Toluol wird unter vermindertem Druck abdestilliert.

Ausbeute: 1172 Teile Bis-$\beta$-ketoamid der Formel

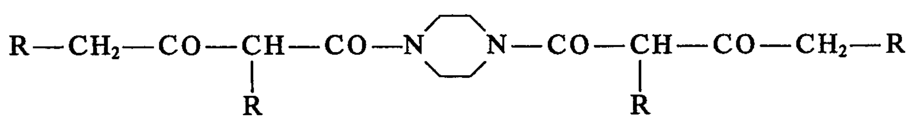

$R = C_{16}H_{33}$

Schmp. 44–45°C
Aminzahl: 0,199 mmol/g
Trockengehalt: 99,3%

Beispiel 9

Es wird wie in Beispiel 8 verfahren, jedoch werden 545 Teile des in Beispiel 2 angegebenen Diketens (= 1 Mol) angewendet.

Ausbeute: 637 Teile Mono-$\beta$-ketoamid der Formel

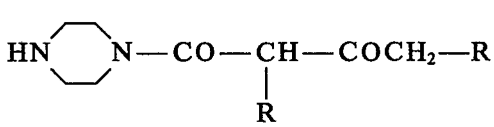

$R = C_{16}H_{33}$

Schmp. 54–56°C
Aminzahl: 0,991 mmol/g
Trockengehalt: 98,1%

Beispiel 10

Es wird wie in Beispiel 8 verfahren, jedoch werden 129 Teile (1 Mol) N-(2-Aminoethyl)-piperazin mit 545 Teilen (= 1 Mol) des in Beispiel 2 verwendeten Diketens angewendet.

Ausbeute: 675 Teile Mono-$\beta$-ketoamid der Formel

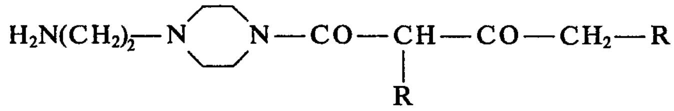

$R = C_{16}H_{33}$

Schmp. 63–66°C
Aminzahl: 3,555 mmol/g
Trockengehalt: 98,8%.

Beispiel 11

In einem Rührgefäß (Rührer, Rückflußkühler, Thermometer) werden 166 Teile (1 Mol) 1,2-Diaminocyclohexan auf 60°C erwärmt und in 15 min 1090 Teile (= 2 Mol) des in Beispiel 2 angegebenen Diketens zugegeben. Dabei steigt die Temperatur auf 92°C. Die Schmelze wird 2 Stunden bei 130°C gerührt, dann ist kein Diketen mehr nachweisbar.

Ausbeute: 1239 Teile Bis-$\beta$-ketoamid der Formel

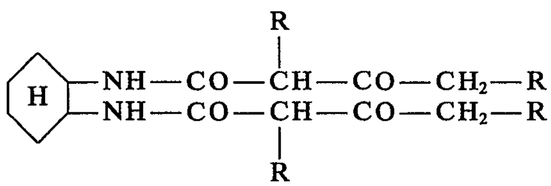

$R = C_{16}H_{33}$

Schmp. 87–89°C
Aminzahl: 0,146 mmol/g.

Beispiel 12

Es wird wie in Beispiel 11 verfahren, jedoch werden 136 Teile (1 Mol) 1,3-Xylylendiamin und 545 Teile (1 Mol) des in Beispiel 2 angegebenen Diketens verwendet. Die Reaktionsdauer war 4 Stunden bei 130°C.

Ausbeute: 677 Teile Mono-$\beta$-ketoamid der Formel

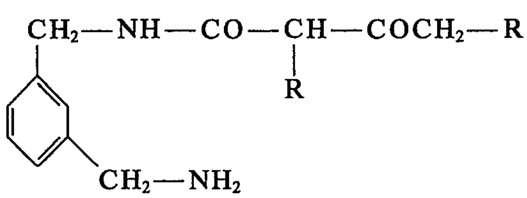

$R = C_{16}H_{33}$

Schmp. 85–87°C
Aminzahl: 0,5 mmol/g
Trockengehalt: 99,4%.

Durch Umsetzung mit 1090 Teilen (= 2 Mol) des in Beispiel 2 angegebenen Diketen erhält man das entsprechende Bis-$\beta$-ketoamid (Reaktionsdauer 8 Stunden bei 130°C).

## Beispiel 13

435 Teile des nach Beispiel 3 erhaltenen Mono-β-ketoamids werden zusammen mit 142 Teilen Stearinsäure unter Stickstoff geschmolzen und 1 Stunde bei 110°C gerührt.

Ausbeute: 575 Teile des Stearats der Formel

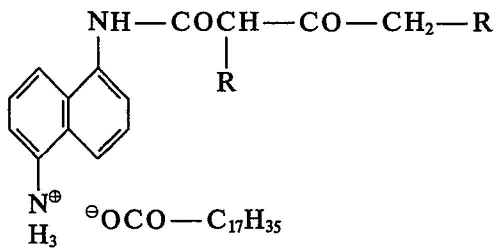

R = $C_{16}$-Alkyl

Schmp. 57°C
Trockengehalt: 98,8%.

## Beispiel 14

374 Teile (0,5 Mol) des nach Beispiel 7 erhaltenen Mono-β-ketoamids werden unter Stickstoff mit 163 Teilen (0,5 Mol) Dodecylbenzolsulfonsäure geschmolzen und 1 Stunde bei 100°C gerührt.

Ausbeute: 549 Teile des Sulfonats der Formel

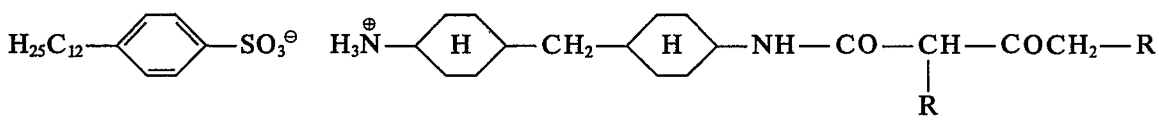

R = $C_{16}H_{33}$

Schmp. 87°C
Trockengehalt: 97,2%.

## Beispiel 15

In einem Rührgefäß (mit Rührer, Rückflußkühler und Thermometer) werden 545 Teile (= 1 Mol) des in Beispiel 2 angegebenen Diketens und 99 Teile 4,4'-Diaminodiphenylmethan (= 0,5 Mol) in 700 Teilen Toluol auf 70°C erwärmt bis kein Diketen mehr nachweisbar ist. Dauer 5 Stunden. Das Toluol wird unter vermindertem Druck abdestilliert (70–90°C).

Ausbeute: 630 Teile Bis-β-ketoamid der Formel

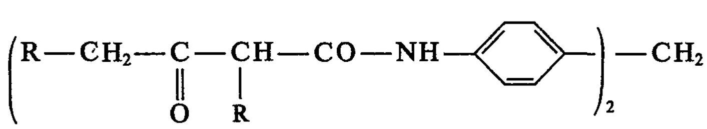

R = $C_{16}H_{33}$

Trockengehalt: 99,1%
Aminzahl: 0.

## Anwendungsbeispiel 1

a) Buntlack: In 50 Teilen Xylol werden 0,5 Teile des nach Beispiel 3 erhaltenen Mono-β-ketoamids in der Wärme gelöst und dann 5 Teile eines feinteiligen agglomerierten Kupferphthalocyanins (erhalten durch 25stündiges Mahlen von kristallisiertem Rohkupferphthalocyanin in Abwesenheit von Mahlhilfsmitteln in der Kugelmühle; das Mahlgut besteht aus 10 bis 200 µm großen Agglomeraten, die aus Primärteilchen ≤ 0,1 µm aufgebaut sind) zugegeben. Die Suspension wird 10 min mit einem Zahnscheibenrührer (Dissolver) mit 12 000 U/min gerührt. Danach gibt man 79 Teile Alkyd/Melaminharzlack (35%ige Lösung in Butanol/Xylol) zu und rührt weitere 10 min mit dem Dissolver.

b1) Zur Bestimmung der Farbstärke werden 1,1 Teile des nach a) erhaltenen Buntlacks mit 5 Teilen Weißlack (20% Titandioxid in Rutilform) homogen gemischt und der erhaltene Weißverschnitt mit einer 150 µm Spiralrakel auf Kontrastblech abgezogen.
Die Färbung wird nach dem Ablüften 30 min bei 130°C eingebrannt.
Die Farbstärke der Färbung wird spektralphotometrisch nach dem FIAF-Programm (L. Gall, »Farbe und Lack« 75 (1969) Seiten 854–862) ermittelt.
Man findet ein Aufhellverhältnis (AV) von 34,36.

b2) Bei einem Vergleich, bei dem das gleiche feinteilige agglomerierte Kupferphthalocyanin jedoch in Abwesenheit des Mono-β-ketoamids in den gleichen Lack eingearbeitet wurde, erhält man eine Weißverschnittfärbung mit einem AV von 20,5.

b3) Die maximal erreichbare Farbstärke (= Endfarbstärke) wird erhalten, wenn man den nach a) erhaltenen Buntlack noch weitere 20 min mit dem Zahnscheibenrührer bei 12 000 U/min rührt und dann 20 min in einer Perlmühle dispergiert. Eine nach b1) hergestellte Weißverschnittfärbung hat ein AV von 37,8, d. h., die Dispergierung in Gegenwart des Mono-β-ketoamids gibt bereits nach 20 min im Dissolver fast die Endfarbstärke.
Ein praktisch gleiches Ergebnis wird mit den Mono-β-ketoamiden der Beispiele 7, 9 und 10 erzielt.
Ähnliche Ergebnisse erhält man mit den Bis-β-ketoamiden der Beispiele 1, 2, 4, 5, 6, 8 und 15.

## Anwendungsbeispiel 2

a) Buntlack: 5 Teile des im Anwendungsbeispiel 1 a) verwendeten feinteiligen, agglomerierten Kupferphthalocyanins werden in eine Lösung, die 0,5 Teile des nach Beispiel 3 hergestellten Mono-β-ketoamids in 10 Teilen Xylol und 79 Teilen Alkyl/Melaminharz-Lack (38%ige Lösung in Butanol/Xylol) enthält, eingetragen und 10 min mit einem Zahnscheibenrührer bei 12 000 U/min dispergiert.

b1) Die Farbstärke wird wie im Anwendungsbeispiel 1 b1) bestimmt; AV = 33,6.

b2) Bei einem Vergleich, bei dem das gleiche Kupferphthalocyanin wie bei a), jedoch in Abwesenheit des Mono-β-ketoamids dispergiert wurde, erhält man eine Weißverschnittfärbung mit einem AV von 16,8.
Gleiche Ergebnisse werden mit den Mono-β-ketoamiden der Beispiele 7, 9 und 10 erhalten.
Ähnliche Ergebnisse werden bei Anwendung der Bis-β-ketoamide der Beispiele 1, 2, 4, 5, 6, 8 und 15 erzielt.

## Anwendungsbeispiel 3

a) Druckfarbe: 12 Teile β-Kupferphthalocyanin in Pigmentform (für Druckfarben) und 1,2 Teile des nach Beispiel 1 erhaltenen Bis-β-ketoamids werden in 138 Teile einer 35%igen Lösung eines modifizierten Kolophoniumharzes (®Albertol KP 670 der Reichhold-Albert Chemie AG) in Toluol mit 300 Teilen Stahlkugeln (2 bis 3 mm ⌀) in einem Polyethylenbecher auf einer Schüttelmühle (®Red Devil) angerieben. Nach 5 min. entnimmt man eine Probe (1) und schüttelt weitere 25 min. Nach 1stündigem Abkühlen werden die Kugeln über ein Sieb abgetrennt.
Die Auslaufzeit der Druckfarbe (DIN-Becher mit 4 mm Düse) beträgt 24 s.

b) Vergleich: das gleiche Pigment wird wie unter a) angegeben jedoch ohne Bis-β-ketoamid zu einer Druckfarbe verarbeitet. Auslaufzeit aus DIN-Becher mit 4 mm Düse 46,3 s.

c1) Mit einer Messerrakel werden mit der nach a) und b) erhaltenen Druckfarben und den Proben 1 Draw-downs auf Pergamentpapier, Illustriertenpapier und auf Acetatfolie hergestellt.

c2) Auswertung der Draw-downs:

c2.1) Die Farbstärke wurde photometrisch ermittelt.

c2.2) Dispergierhärte: aus dem Farbstärkenverhältnis der Druckfarbe und der Probe 1 (Dispergierdauer 30 bzw. 5 min) wird die Dispergierhärte DH nach folgender Formel ermittelt

$$DH = \left(\frac{\text{Farbstärke } 30'}{\text{Farbstärke } 5'} - 1\right) \cdot 100.$$

c2.3) An den Draw-downs auf Acetatfolie und Illustriertenpapier wurde der Glanz mit Hilfe des Glanzmeßgeräts der Fa. Gardener gegen einen Schwarzglasstandard bei einem Winkel von 45° gemessen.

Die nach c2.1) bis c2.3) erhaltenen Meßergebnisse sind in den folgenden Tabellen zusammengestellt:

Tabelle Ia)

| | Farbstärke [%] | Dispergierhärte (DH) | Auslaufzeit [s] |
|---|---|---|---|
| Druckfarbe a) | 110 | 0 | 24 |
| Vergleich b2) | 100 | 18 | 46,3 |

Tabelle Ib)

| | Glanz auf Acetatfolie, Illustriertenpapier nach Dispergierdauer | | | |
|---|---|---|---|---|
| | 5′ | 30′ | 5′ | 30′ |
| Druckfarbe a) | 88% | 93% | 46% | 38% |
| Vergleich b2) | 41% | 80% | 30% | 36% |

Praktisch gleiche Ergebnisse werden mit den $\beta$-Ketoamiden der Beispiele 2 bis 10 erhalten.

Anwendungsbeispiel 4

a) Es wird wie beim Anwendungsbeispiel 3a) verfahren, jedoch verwendet man das im Anwendungsbeispiel 1a) angegebene feinteilige agglomerierte Kupferphthalocyanin.
b) Der Vergleich wird aus dem gleichen Kupferphthalocyanin jedoch ohne Bis-$\beta$-ketoamid hergestellt.
c) Die Färbungen werden wie im Anwendungsbeispiel 3c angegeben hergestellt und ausgewertet.

Die Ergebnisse sind in der Tabelle II zusammengestellt.

| | Farbstärke | DH | Auslaufzeit | Glanz auf Acetat, | | Illustrierten-papier | |
|---|---|---|---|---|---|---|---|
| | | | | nach Dispergierdauer | | | |
| | [%] | | [s] | 5′ [%] | 30′ [%] | 5′ [%] | 30′ [%] |
| Druckfarbe a) | 108 | 11 | 36 | 87 | 92 | 43 | 46 |
| Vergleich b) | 100 | 18 | tixotrop | 52 | 94 | 35 | 27 |

Entsprechende Ergebnisse werden erhalten, wenn als Bis-$\beta$-ketoamide die der Beispiele 2, 4, 5, 6, 7 oder 8 oder Mono-$\beta$-ketoamide der Beispiele 3, 7, 9, 10, 13 oder 14 verwendet werden.

Anwendungsbeispiel 5

a) 7 Teile des im Anwendungsbeispiel 1 a) angegebenen Kupferphthalocyanins und 0,7 Teile des Bis-$\beta$-ketoamids des Beispiels 15 werden in eine Mischung aus 37,8 Teilen Alkydharz in 5,2 Teilen Mineralöl eingerührt und 5 min bei 12 000 U/min bei 95°C mit einem Zahnscheibenrührer vordispergiert. Dann gibt man 200 Teile Stahlkugeln (⌀: 3 mm) zu und dispergiert 5 min. Man entnimmt eine Probe (1) und dispergiert dann weitere 15 min. Nach Entnahme einer Probe (2) wird noch 20 min. dispergiert.

b) Vergleich: es wird wie unter a), jedoch ohne Bis-$\beta$-ketoamid gearbeitet.

c) Zur Farbstärkenbestimmung werden die Proben mit Weißpaste gemischt und an den erhaltenen Färbungen das AV bestimmt. Die Ergebnisse sind in der Tabelle III zusammengestellt:

| | AV nach Dispergierdauer | | |
|---|---|---|---|
| | 5 | 20 | 40 min |
| Druckfarbe a) | 29,25 | 35,35 | 35,88 |
| Vergleich b) | 20,15 | 31,57 | 35,70 |

D. h., das Pigment wird durch den Zusatz an Bis-$\beta$-ketoamid in der Dispergierbarkeit und in der Farbstärkenentwicklung verbessert.

Entsprechende Ergebnisse erhält man, wenn $\beta$-Ketoamide nach einem der Beispiele 1 bis 10 oder 13 oder 14 verwendet werden.

Anwendungsbeispiel 6

a) 8 Teile $\beta$-Kupferphthalocyanin/Pigmentform (für Druckfarben) und 0,5 Teile des nach Beispiel 15 erhaltenen Bis-$\beta$-ketoimids werden in eine Mischung aus 17,5 Teilen Nitrocellulose in 62,5 Teilen Essigester und 15,5 Teilen Ethanol eingetragen und nach dem Zugeben von Stahlkugeln 10 min auf einer Schüttelmühle ($^R$ Red Devil) angerieben. Nach dem Ziehen einer Probe wird weitere 50 min dispergiert. Nach dem Abkühlen (1 Stunde) werden die Kugeln abgetrennt und die Druckfarbe bei Raumtemperatur über Nacht gelagert.

b) Vergleich: es wird wie unter a) gearbeitet, jedoch ohne Zusatz an Bis-$\beta$-ketoamid.

c) Von beiden Druckfarben wird die Auslaufzeit im DIN-Becher mit 4 mm-Düse bestimmt.

| | Auslaufzeit [sec] |
|---|---|
| Druckfarbe a) | 22,1 |
| Vergleich b) | 30,5 |

Der Zusatz bewirkte eine deutliche Verbesserung des Fließvermögens.

Ähnliche Ergebnisse werden erhalten, wenn man das Bis-$\beta$-ketoamid durch die Ketoamide nach einem der Beispiele 1 bis 10, 13 oder 14 ersetzt.

Anwendungsbeispiel 7

a) Buntlack: In 50 Teilen Xylol werden 0,5 Teile des nach Beispiel 13 erhaltenen Salzes des Mono-$\beta$-ketoamids in der Wärme gelöst und dann 5 Teile eines feinteiligen agglomerierten Kupferphthalocyanins (erhalten durch 25stündiges Mahlen von kristallisiertem Rohkupferphthalocyanin in Abwesenheit von Mahlhilfsmitteln in der Kugelmühle; das Mahlgut besteht aus 10 bis 200 $\mu$m großen Agglomeraten, die aus Primärteilchen $\leq$ 0,1 $\mu$m aufgebaut sind) zugegeben. Die Suspension wird 10 min mit einem Zahnscheibenrührer (Dissolver) mit 12 000 U/min gerührt. Danach gibt man 79 Teile Alkyd/Melaminharzlack (35%ige Lösung in Butanol/Xylol) zu und rührt weitere 10 min mit dem Dissolver.

b1) Zur Bestimmung der Farbstärke verfährt man nach Anwendungsbeispiel 1 b1). Man ermittelt ein Aufhellverhältnis von 35,92.

b2) Bei einem Vergleich, bei dem das gleiche feinteilige agglomerierte Kupferphthalocyanin jedoch in Abwesenheit des Mono-$\beta$-ketoamids in den gleichen Lack eingearbeitet wurde, erhält man eine Weißverschnittfärbung mit einem AV von 20,5.

b3) Die maximal erreichbare Farbstärke (= Endfarbstärke) wird erhalten, wenn man den nach a) erhaltenen Buntlack noch weitere 20 min mit dem Zahnscheibenrührer bei 12 000 U/min rührt und dann 20 min in einer Perlmühle dispergiert. Eine nach b1) hergestellte Weißverschnittfärbung hat ein AV von 37,8, d. h., die Dispergierung in Gegenwart des Mono-$\beta$-ketoamids gibt bereits nach 20 min im Dissolver fast die Entfarbstärke.
Ein praktisch gleiches Ergebnis wird mit dem Dodecylbenzolsulfonat des Mono-$\beta$-ketoamids des Beispiels 14 erzielt.

Anwendungsbeispiel 8

a) In 95 Teilen eines Buch-/Offset-Firnis werden 0,5 Teile des Bis-$\beta$-ketoamids aus Beispiel 4 und 5 Teilen eines feinteiligen, agglomerierten Kupferphthalocyanins zunächst 10 min bei 50°C mit einem Zahnscheibenrührer mit 1 200 U/min gerührt. Man entnimmt eine Probe (1) und rührt weitere 10 min bei 1 200 U/min. Nach dem Entnehmen einer zweiten Probe (2) wird nochmals 10 min bei 1 200 U/min gerührt (Probe 3).

b1) Die Farbstärkenbestimmung erfolgt nach Anwendungsbeispiel 1, b1) durch Mischen mit Standardweißpaste und photometrischer Auswertung der Weißverschnitte.

Es wurden folgende Aufhellverhältnisse bestimmt

| | Probe 1 (10 min) | Probe 2 (20 min) | Probe 3 (30 min) |
|---|---|---|---|
| AV | 6,79 | 6,99 | 7,77 |

b2) Zum Vergleich wird das gleiche feinteilige agglomerierte Kupferphthalocyanin im gleichen Firnis in gleicher Weise angerieben und daraus Weißverschnitte hergestellt. Es wurden folgende Aufhellverhältnisse ermittelt

Vergleich

| | Probe 1 (10 min) | Probe 2 (20 min) | Probe 3 (30 min) |
|---|---|---|---|
| AV | 4,73 | 6,34 | 6,57 |

D. h., das Bis-$\beta$-ketoamid bewirkt eine deutlich bessere Dispergierbarkeit und eine deutliche höhere Endfarbstärke.

Beispiel 16

Es wird wie in Beispiel 1 verfahren, jedoch werden 214 Teile (= 1 Mol) 4,4'-Diaminodiphenylether (98%ig) und 545 Teile Bis-$C_{16}$-alkyldiketen (mittleres M. 542; Gehalt: 99,4% = 542 Teile 100% = 1 Mol) in 600 Teilen Toluol verwendet.

Ausbeute: 795 Teile (= 98,5% der Theorie) Monoketoamid der Formel

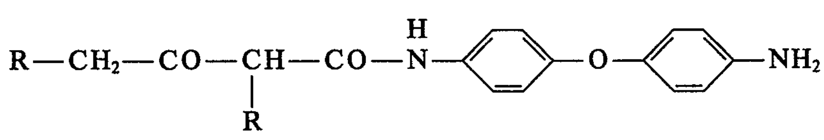

mit R = $C_{16}H_{33}$

in Form einer Schmelze. Schmp. 87°C, Aminzahl:
ber. 1,33 mMol/g; gef. 1,084 mMol/g (Titration mit Perchlorsäure in Eisessig); Trockengehalt: 93,1%.

Bei der Anwendung des erhaltenen Monoketoamids nach dem Anwendungsbeispiel 2 erhält man Weißverschnitte, die eine hohe Farbstärke aufweisen.

In Gegenwart des Ketoamids erhält man Druckfarben mit sehr gutem Fließverhalten, hoher Farbstärke, die Färbungen mit hoher Brillanz und hohem Glanz liefern.

Beispiel 17

Es wird wie in Beispiel 1 verfahren, jedoch werden 214 Teile (= 1 Mol) 4,4'-Diaminodiphenylether (98%ig) und 1090 Teile des in Beispiel 15 angegebenen Diketens (= 2 Mol) in 800 Teilen Toluol verwendet. Ausbeute: 1366 Teile (= 99,6% der Theorie).

Bisketoamid der Formel

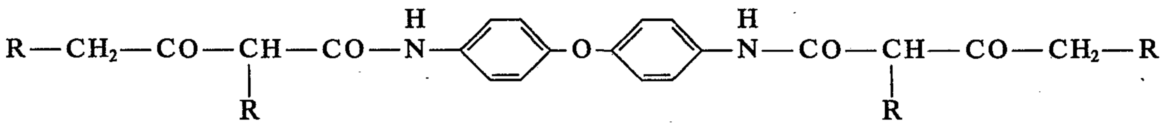

mit R = $C_{16}H_{33}$ in Form einer Schmelze.

Schmp. 88°C, Aminzahl: 0 (Titration mit Perchlorsäure in Eisessig), Trockengehalt: 94,3%.

In Gegenwart des Bisketoamids erhält man Druckfarben (hergestellt nach Anwendungsbeispiel 3) die eine geringe Viskosität und hohe Farbstärke aufweisen. Mit den Farben werden Drucke mit hohem Glanz erhalten.

**Patentansprüche**

1. $\beta$-Ketoamide der allgemeinen Formel

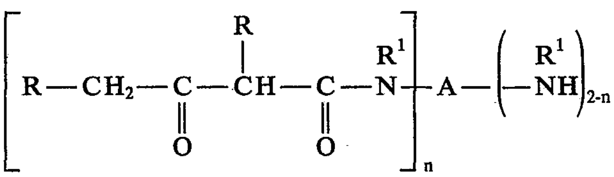

in der R für $C_8$- bis $C_{18}$-Alkyl oder $C_8$- bis $C_{18}$-Alkenyl, $R^1$ für Wasserstoff und —A— für 1,5-Naphthylen, 1,8-Naphthylen, 1,3-Phenylen, 1,4-Phenylen, für gegebenenfalls in 3,3'- oder in 2,2'-Stellung durch Chlor, Methyl oder Methoxy substituiertes 4,4'-Diphenylen, 4,4'-Diphenylenether, für gegebenenfalls in 3,3'-Stellung durch Isopropyl oder Methoxy und gegebenenfalls in 5,5'-Stellung durch Isopropyl substituiertes 4,4'-Diphenylenmethan oder 4,4'-Dicyclohexylenmethan, für gegebenenfalls in 3,3'-Stellung durch Isopropyl oder Methoxy substituiertes 4-Cyclohexylen-4'-phenylenmethan, für 2,2-Bi(phenylen-4')-propan, 2,2-Bis(cyclohexylen-4')propan, 2-(Cyclohexylen-4')-2-(phenylen-4")-propan, 1,2-Cyclohexylen, 1,3-Bismethylenphenyl, 4,4'-Diphenylenamin, Di-(3-methylphenylen-4)-methan, 3-Methyl-cyclohexylen-4-3'-methylphenylen-4'-methan, Bis-(3-methyl-cyclohexylen-4)-methan oder

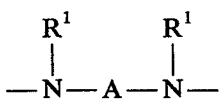 für 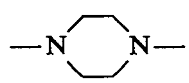

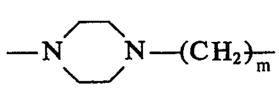

oder

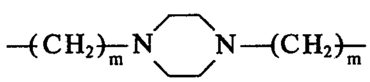

n für 1 oder 2 und

m für 2 oder 3 stehen

und — wenn n = 1 ist — deren Salze von $C_{10}$- bis $C_{20}$-Alkansäuren, $C_{10}$- bis $C_{20}$-Alkensäuren, $C_{10}$- bis $C_{20}$-Alkansulfonsäuren, $C_{10}$- bis $C_{20}$-Alkensulfonsäuren oder $C_1$- bis $C_{20}$-Alkylbenzolsulfonsäuren.

2. $\beta$-Ketoamide gemäß Anspruch 1, dadurch gekennzeichnet, daß $R^1$ = H und —A— 1,5-Naphthylen, gegebenenfalls in 3,3'- oder in 2,2'-Stellung durch Chlor, Methyl oder Methoxy substituiertes Diphenylen-4,4', Di(phenylen-4)-methan, 2,2-Bis(phenylen-4')-propan, Di-(3-methylphenylen-4)-methan, 3-Methyl-cyclohexylen-4-3'-methylphenylen-4'-methan, Bis(cyclohexylen-4)-methan, Bis-(3-methylcyclohexylen-4)-methan, 2,2-Bis(cyclohexylen-4')-propan, 4,4'-Diphenylenether oder

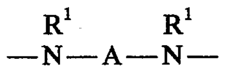

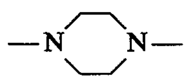

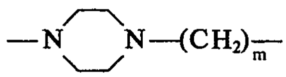

oder

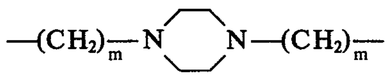

und m = 2 oder 3 ist.

3. $\beta$-Ketoamide gemäß Anspruch 1 oder 2, dadurch gekennzeichnet, daß R $C_{10}$- bis $C_{16}$-Alkyl oder $C_{10}$- bis $C_{16}$-Alkenyl ist.

4. $\beta$-Ketoamide gemäß Anspruch 1 oder 2, dadurch gekennzeichnet, daß R $C_{10}H_{21}$-Alkyl, $C_{14}H_{29}$-Alkyl, $C_{16}H_{33}$-Alkyl oder $C_{16}H_{31}$-Alkenyl ist.

5. Pigmentzubereitung, dadurch gekennzeichnet, daß diese (a) ein feinteiliges Pigment, das sich vom Anthrachinon oder davon abgeleiteten kondensierten Ringsystemen, von der Perylen-3,4,9,10-tetracarbonsäure, von Chinophthalonen, Dioxazinen, Chinacridon oder vom Phthalocyanin ableitet, und (b) mindestens ein $\beta$-Ketoamid gemäß Anspruch 1, 2, 3 oder 4 enthält.

6. Zubereitung gemäß Anspruch 5, dadurch gekennzeichnet, daß diese 2 bis 20 Gew.-% (b), bezogen auf (a), enthält.

7. Verwendung der Zubereitung gemäß Anspruch 5 oder 6 zum Herstellen von Druck- und Lackfarben.

## Claims

1. A $\beta$-ketoamide of the general formula

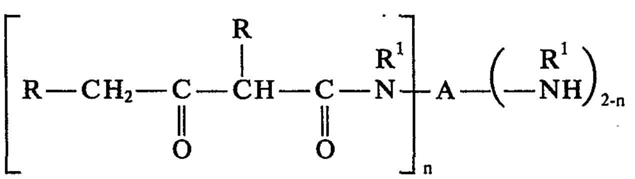

where R is $C_8$—$C_{18}$-alkyl or $C_8$—$C_{18}$-alkenyl, $R^1$ is hydrogen, and —A— is 1,5-naphthylene, 1,8-naphthylene, 1,3-phenylene or 1,4-phenylene; 4,4'-diphenylene ether, or 4,4'-diphenylene which is optionally substituted in the 3,3'- or 2,2'-position by chlorine, methyl or methoxy; 4,4'-diphenylene methane or 4,4'-dicyclohexylene methane which is optionally substituted in the 3,3'-position by isopropyl or methoxy and optionally substituted in the 5,5'-position by isopropyl; 4-cyclohexylene-4'-phenylene-methane optionally substituted in the 3,3'-position by isopropyl or methoxy; 2,2-bis(4'-phenylene)-propane, 2,2-bis(4'-cyclohexylene)-propane, 2-(4'-cyclohexylene)-2-(4''-phenylene)-propane, 1,2-cyclohexylene, 1,3-bismethylene-phenyl, 4,4'-diphenyleneamine, di-(3-methyl-4-phenylene)-methane, 3-methyl-4-cyclohexylene-3'-methyl-4'-phenylene-methane, bis-(3-methyl-4-cyclohexylene)-methane, or

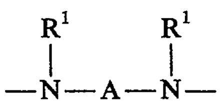

is

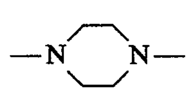

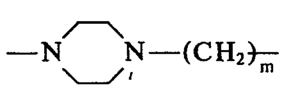

or

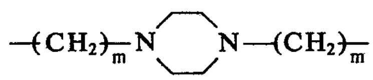

where m is 2 or 3, and n is 1 or 2, and, where n is 1, its salts with $C_{10}$—$C_{20}$-alkanoic acids, $C_{10}$—$C_{20}$-alkenoic acids, $C_{10}$—$C_{20}$-alkanesulfonic acids, $C_{10}$—$C_{20}$-alkenesulfonic acids or $C_1$—$C_{20}$-alkylbenzenesulfonic acids.

2. A $\beta$-ketoamide as claimed as claimed in claim 1, where $R^1$ is hydrogen and —A— is 1,5-naphthylene or is 4,4'-diphenylene which is optionally substituted in the 3,3'- or 2,2'-position by chlorine, methyl or methoxy, or is di-(4-phenylene)-methane, 2,2-bis-(4'-phenylene)-propane, di-(3-methyl-4-phenylene)-methane, 3-methyl-4-cyclohexylene-3'-methyl-4'-phenylene-methane, bis-(4-cyclohexylene)-methane, bis-(3-methyl-4-cyclohexylene)-methane, 2,2-bis-(4'-cyclohexylene)-propane or 4,4'-diphenylene ether, or

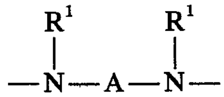 is 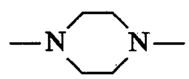

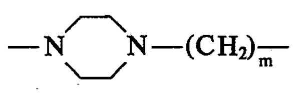

or

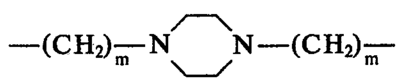

where m is 2 or 3.

3. A $\beta$-ketoamide as claimed in claim 1 or 2, where R is $C_{10}$—$C_{16}$-alkyl or $C_{10}$—$C_{16}$—alkenyl.

4. A $\beta$-ketoamide as claimed in claim 1 or 2, where R is $C_{10}H_{21}$-alkyl, $C_{14}H_{29}$-alkyl or $C_{16}H_{33}$-alkyl or $C_{16}H_{31}$-alkenyl.

5. A pigment formulation containing (a) a finely divided pigment derived from anthraquinone or a fused ring system derived from anthraquinone, or from perylene-3,4,9,10-tetracarboxylic acid, a quinophthalone, a dioxazine, quinacridone or phthalocyanine, and (b) at least one $\beta$-ketoamide as claimed in claim 1, 2, 3 or 4.

6. A formulation as claimed in claim 5, containing from 2 to 20% by weight, based on (a), of (b).

7. The use of a formulation as claimed in claim 5 or 6 for the production of printing inks and surface coatings.

**Revendications**

1. $\alpha$-céto-amides de formule générale

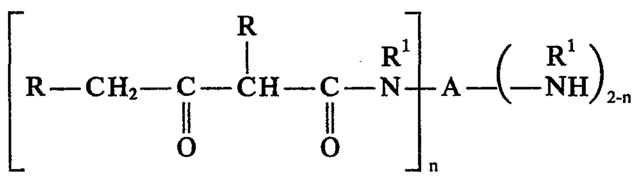

dans laquelle R est mis pour un alcoyle en $C_8$ à $C_{18}$ ou un alcényle en $C_8$ à $C_{18}$,
$R^1$ pour un hydrogène et
—A— pour un 1,5-naphthylène, un 1,8-naphthylène, un 1,3-phénylène, un 1,4-phénylène, pour un 4,4'-diphénylène éventuellement substitué en position 3,3' ou 2,2' par un chlore, un méthyle ou un méthoxy, un éther 4,4'-diphénylénique, pour un 4,4'-diphénylène-méthane éventuellement substitué en position 3,3'- par un isopropyle ou un méthoxy et éventuellement en position 5,5' par un isopropyle ou pour un 4,4'-dicyclohexylène-méthane, pour un 4-cyclohexylène-4'-phénylène-méthane éventuellement substitué en position 3,3' par un isopropyle ou un méthoxy, pour un 2,2'-bis-(phénylène-4')-propane, un 2,2-bis-(cyclohexylène-4')-propane, un 2-(cyclohexylène-4')-2-(phénylène-4")-propane, un 1,2-cyclohexylène, un 1,3-bis-méthylène-phényle, une 4,4'-diphénylène-amine, un di-(3-méthylphénylène-4)-méthane, un 3-méthyl-cyclohexylène-4-3'-méthylphénylène-4'-méthane, un bis-(3-méthyl-cyclohexylène-4)-méthane ou

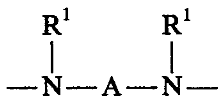 est mis pour 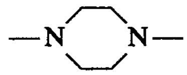

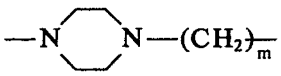

ou

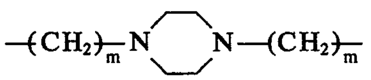

n étant mis pour 1 ou 2 et m pour 2 ou 3, et — lorsque n = 1 — leurs sels d'acides alcanoïques en $C_{10}$ à $C_{20}$, d'acides alcénoïques en $C_{10}$ à $C_{20}$, d'acides alcane-sulfoniques en $C_{10}$ à $C_{20}$, d'acides alcène-sulfoniques en $C_{10}$ à $C_{20}$ ou d'acides alcoyl-benzène-sulfoniques en $C_1$ à $C_{20}$.

2. $\beta$-céto-amides selon la revendication 1, caractérisés en ce que $R^1$ = H et —A— est un 1,5-naphthylène, un diphénylène éventuellement substitué en position 3,3' ou 2,2' par un chlore, un méthyle ou un méthoxy, un di-(phénylène-4)-méthane, un 2,2-bis-(phénylène-4')-propane, un di-(3-méthylphénylène-4)-méthane, un 3-méthyl-cyclohexylène-4,3'-méthylphénylène-4'-méthane, un bis-(cyclohexylène-4)-méthane, un bis-(3-méthyl-cyclohexylène-4)-méthane, un 2,2-bis-(cyclohexylène-4')-propane, un éther 4,4'-diphénylénique ou

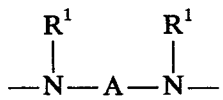 est mis pour 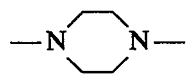

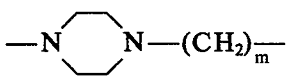

ou

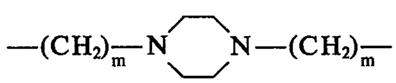

et m = 2 ou 3.

3. $\beta$-céto-amides selon la revendication 1 ou 2, caractérisés en ce que R est un alcoyle en $C_{10}$ à $C_{16}$ ou un alcényle en $C_{10}$ à $C_{16}$.

4. $\beta$-céto-amides selon la revendication 1 ou 2, caractérisés en ce que R est un alcoyle $C_{10}H_{21}$, un alcoyle $C_{14}H_{29}$, un alcoyle $C_{16}H_{33}$ ou un alcényle $C_{16}H_{31}$.

5. Préparation pigmentaire, caractérisée en ce qu'elle contient (a) un pigment finement divisé qui dérive de l'anthraquinone ou de systèmes de noyaux conjugués issus de celle-ci, de l'acide pérylène-3,4,9,10-tétracarboxylique, de quinophthalones, de dioxazines, de la quinacridone ou de la phthalocyanine et (b) au moins un $\beta$-céto-amide selon l'une quelconque des revedications 1 à 4.

6. Préparation selon la revendication 5, caractérisée en ce qu'elle contient 2 à 20% en poids de (b) par rapport à (a).

7. Utilisation de la préparation selon la revendication 5 ou 6 pour la fabrication d'encres d'imprimerie et de peintures-émail.